# FASCICULE DE BREVET EUROPEEN

(11) **EP 1 980 238 B1**
(45) Date de publication et mention de la délivrance du brevet: **20.03.2019**
(21) Numéro de dépôt: 08153896.9
(22) Date de dépôt: 01.04.2008
(51) Int. Cl.: A61K 8/81, A61K 8/37, A61Q 1/02

(54) **Composition cosmétique comprenant une phase continue huileuse**
Kosmetische Zusammensetzung mit kontinuierlicher Ölphase
Cosmetic composition comprising a continuous oily phase

(30) Priorité: 13.04.2007 FR 0754460
(43) Date de publication de la demande: 15.10.2008
(73) Titulaire: L'Oréal, 75008 Paris (FR)
(72) Inventeur: Clavel, Euriel, 75013 Paris (FR); Arnaud, Pascal, 94240 L'Hay Les Roses (FR)
(74) Mandataire: Boulard, Denis

(56) Documents cités:
- WO-A-00/59456
- WO-A1-2007/011449
- US-A- 4 795 631

## Description

La présente invention a pour objet une composition cosmétique de maquillage ou de soin de la peau comprenant une phase continue huileuse, des charges et des huiles spécifiques. L'invention a également pour objet un procédé de maquillage ou de soin de la peau d'être humain comprenant l'application de la composition sur la peau.

La composition de maquillage de la peau peut être un fond de teint, un fard à paupières, un fard à joue, un produit anticernes, un produit de maquillage du corps. Plus spécialement, l'invention porte sur une composition de fond de teint.

La composition est de préférence une composition de maquillage de la peau.

Les compositions de fond de teint sont couramment employées pour apporter une couleur esthétique à la peau, notamment au visage, mais également pour camoufler les imperfections de la peau telles que les rougeurs, les taches, ou encore conférer à la peau un aspect mat par absorption du sébum.
Pour se faire, elles contiennent une phase pulvérulente constituée de pigments et de charges. Plus la teneur en pigments et en charges dans la composition est importante, meilleur sera le camouflage des défauts de couleur de la peau, ou l'absorption du sébum. Cependant, une teneur trop importante en pigments et charges entraîne souvent le dessèchement des matières kératiniques, ce qui provoque pour l'utilisatrice une sensation de tiraillement, rendant ainsi le maquillage inconfortable à porter pendant la journée.

Il est connu d'introduire, dans les compositions cosmétiques, des huiles ou des corps gras pour diminuer la sensation de dessèchement à l'application et pour améliorer le confort du maquillage. Cependant, l'introduction de corps gras liquides ou pâteux dans les compositions de maquillage présente l'inconvénient de laisser sur la peau une sensation de gras, voir de collant au toucher, ce qui les rend peu attrayantes pour les utilisatrices.

Il existe donc un besoin pour des compositions de maquillage ou de soin de la peau permettant un camouflage satisfaisant des imperfections de couleur et/ou de relief de la peau, une bonne absorption du sébum, sans pour autant dessécher la peau ou à l'inverse conférer à l'utilisatrice une sensation de gras voire de collant à l'application ou au cours de la journée.

Le demandeur a découvert qu'il était possible d'obtenir une telle composition en associant des particules de polyméthacrylate de méthyle et des huiles ester de dipentaérythritol dans une composition comprenant une phase continue huileuse.

Les compositions obtenues présentent, en outre, de bonnes propriétés de douceur et d'étalement lors de leur application sur la peau.

Selon un premier aspect, la présente invention a pour objet une composition de maquillage ou de soin de la peau comprenant une phase continue huileuse et une phase particulaire, ladite phase continue huileuse comprenant au moins un ester de dipentaérythritol, et ladite phase particulaire comprenant des particules de polymère acrylique.

Selon un second aspect, la présente invention a pour objet un procédé de maquillage et/ou de soin de la peau comprenant l'application sur ladite peau d'une composition telle que décrite précédemment.

L'invention a encore pour objet, selon un troisième aspect l'utilisation d'une composition telle que décrite précédemment, pour obtenir un maquillage permettant une bonne absorption du sébum, sans dessécher la peau.

Enfin l'invention a pour objet l'utilisation d'une composition telle que décrite précédemment, pour obtenir un maquillage confortable pour l'utilisatrice, ne conférant pas de sensation de gras voire de collant à l'application ou au cours de la journée.

### Les particules de polymères acryliques

La composition selon l'invention comprend des particules de polymères acryliques.
On entend par « particules de polymère acrylique » des charges obtenues par polymérisation d'un monomère acrylate ou méthacrylate d'alkyle, sous la forme d'un homopolymère ou d'un copolymère. A titre illustratif non limitatif, on peut notamment citer les particules de polyméthacrylate de méthyle, de polyméthacrylate de méthyle/diméthacrylate d'éthylène glycol, de polyméthacrylate d'allyle/diméthacrylate d'éthylène glycol et de copolymère diméthacrylate d'éthylène glycol/méthacrylate de lauryle.

En particulier, ces particules ne sont pas filmogènes, c'est-à-dire qu'elles ne forment pas un film continu lorsqu'elles sont déposées sur un support tel que la peau.

Les particules de polymères acryliques se présentent généralement sous la forme de particules sphériques creuses ou pleines, de couleur blanche dont la taille moyenne en nombre est généralement à l'échelle du micromètre, en particulier varie de 3 à 20 microns et généralement varie de 7 à 15 microns. Par « taille moyenne en nombre », on désigne la dimension donnée par la distribution granulométrique statistique à la moitié de la population, dite D50.

A titre représentatif et non limitatif des particules de polymères acryliques convenant à l'invention, on peut notamment citer :
- les particules de polyméthyméthacrylate réticulé comme par exemple le "COVABEAD LH85" commercialisé par la société LC WACKHERR ou non réticulé comme le SJ TOUCH 1 commercialisés par la société NIHON JUNYAKU,
- les particules de copolymère méthacrylate de méthyle / acrylate de butyle vendu sous la dénomination de SEPIPRESS M par la société SEPPIC,
- les particules de copolymère acrylate de méthyle / éthylène vendu sous la dénomination de EMAA par la société KOBO PRODUCTS Inc.,
- les particules de copolymère réticulé méthacrylate de méthyle / di méthacrylate d'éthylène glycol vendu sous la dénomination de GANZPEARL GMP 0820 par la société GANZ CHEMICAL, sous la dénomination de TECHPOLYMER MBP-8 par la société SEKISUI PLASTICS ou encore sous la dénomination de SUNPMMA-S par la société SUNJIN CHEMICAL,
- les particules de polyméthacrylate de méthyle / di méthacrylate d'éthylène glycol, comme par exemple le "DOW CORNING 5640 MICROSPONGE SKIN OIL ADSORBER" commercialisé par la société DOW CORNING,
- les particules de copolymère réticulé méthacrylate de méthyle / di méthacrylate d'éthylène glycol vendu sous la dénomination de GANZPEARL PM 030 par la société GANZ CHEMICAL,
- les particules de polyméthacrylate d'allyle/di méthacrylate d'éthylène glycol, comme par exemple le "POLY-PORE L200" ou le "POLY-PORE E200" commercialisés par la société AMCOL ,
- les particules de copolymère méthacrylate de lauryle / di méthacrylate d'éthylène glycol, comme par exemple le "POLYTRAP 6603" commercialisé de la société DOW CORNING.

Les particules de polymères acryliques selon l'invention peuvent être présentes dans la composition en une quantité allant de 0,1 à 10 % en poids, par rapport au poids total de la composition, en particulier de 2 à 7 % et plus particulièrement de 3 à 6% en poids.

Dans le cadre de la présente invention, les particules de polymères acryliques sont avantageusement formulées dans la phase continue huileuse de la composition selon l'invention.

### Esters de dipentaérythritol

La phase continue huileuse de la composition selon l'invention comprend au moins un ester de dipentaérythritol.
Les esters de dipentaérythritol selon l'invention sont obtenus par estérification totale du dipentaérythritol par des acides linéaires ou ramifiés, saturés ou insaturés comportant de 3 à 24 atomes de carbone, de préférence de 4 à 18 atomes de carbone et de manière encore plus préférentielle de 5 à 16 atomes de carbone.
On entend par estérification totale, l'absence totale de fonction hydroxy résiduelle.
Les acides utilisés pour estérifier le dipentaérythritol peuvent être utilisés sous la forme de mélange.
Les esters de l'invention sont sous forme liquides à température ambiante (25°C).

De manière non limitative, on peut citer à titre d'exemple des esters de dipentaérythritol utilisés selon la présente invention :
- l'héxacaprylate/caprate de dipentaérythrityle vendu sous la dénomination de DUB DPHCC par la société STEARINERIE DUBOIS,
- l'héxa C5-9 carboxylate de dipentaérythrityle vendu sous la dénomination de LexFeel 350 par la société INOLEX CHEMICAL COMPANY.

Selon un mode préféré de réalisation, l'ester de dipentaérythritol préféré est l'héxacaprylate/caprate de dipentaérythrityle.

Les esters de dipentaérythritol peuvent être présents dans la composition en une quantité allant de 0,1 à 30% en poids par rapport au poids total de la composition, en particulier de 1 à 20% et plus particulièrement de 3 à 15% en poids.

### Phase grasse

Outre l'ester de dipentaétrythritol, la phase huileuse de la composition selon l'invention peut comprendre au moins une huile additionnelle, volatile ou non volatile.

Par « huile volatile », on entend une huile susceptible de s'évaporer de la peau, en moins d'une heure à température ambiante et pression atmosphérique. Cette huile présente notamment une pression de vapeur, à température ambiante (25 °C) et pression atmosphérique (760 mm Hg) allant de 0,13 Pa à 40 000 Pa (10⁻³ à 300 mm de Hg), notamment allant de 1,3 Pa à 13 000 Pa (0,01 à 100 mm de Hg), et en particulier allant de 1,3 Pa à 1300 Pa (0,01 à 10 mm de Hg).
En outre, l'huile volatile a généralement un point d'ébullition, mesuré à pression atmosphérique, allant de 150 °C à 260 °C, et notamment allant de 170°Cà250°C.

Avantageusement, l'huile volatile contient une ou plusieurs huiles organiques volatiles dont le point éclair va de 30 °C à 102 °C, en particulier de 40 °C à 55 °C, et notamment de 40 °C à 50 °C et leurs mélanges.

Par « huile non volatile », on entend tout milieu susceptible de rester sur la peau pendant plusieurs heures. Une huile non volatile à en particulier une pression de vapeur à température ambiante et pression atmosphérique, non nulle inférieure à 0,001 mm de Hg (0,13 Pa).
La phase grasse liquide (ou phase huileuse) de l'émulsion selon l'invention contient au moins une huile hydrocarbonée volatile, qui selon le premier aspect de l'invention est au moins de l'isohéxadécane.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

Comme huile volatile utilisable dans l'invention, on peut citer les huiles volatiles hydrocarbonées ayant de 8 à 16 atomes de carbone, linéaires ou ramifiées, et leurs mélanges et notamment les alcanes ramifiés en C₈-C₁₆ comme les isoalcanes (appelées aussi isoparaffines) en C₈-C₁₆, l'isododécane, l'isohexadécane et par exemple les huiles vendues sous les noms commerciaux d'Isopars® ou de Permethyls®, les esters ramifiés en C₈-C₁₆ comme le néo pentanoate d'iso-hexyle et leurs mélanges. On utilise en particulier l'isohexadécane.
Les huiles volatiles peuvent également être siliconées comportant éventuellement des groupes alkyles ou alkoxy pendants, ou en bout de chaîne siliconée, les huiles volatiles fluorées, et leurs mélanges.

Comme huile siliconée volatile utilisable dans l'invention, on peut citer les huiles de silicones linéaires, ramifiées ou cycliques ayant une viscosité à température ambiante inférieure à 8 mm²/s et ayant notamment de 2 à 7 atomes de silicium, ces silicones comportant éventuellement des groupes alkyle ou alkoxy ayant de 1 à 10 atomes de carbone pendants ou au bout de chaque silicone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.
L'huile volatile fluorée n'a généralement pas de point éclair.

Comme huile volatile fluorée, on peut citer le nonafluoroéthoxybutane, le nonafluorométhoxybutane, le décafluoropentane, le tétradécafluorohexane, le dodécafluoropentane, et leurs mélanges.

L'huile volatile additionnelle peut être présente en une teneur allant de 1 % à 80 % en poids par rapport au poids total de la composition, de préférence de 2 % à 50 % en poids et de manière encore plus préférentielle de 3 à 30 %.

La composition peut comprendre, outre l'ester de dipentaérythritol, au moins une autre huile non volatile.
Comme huiles non volatiles utilisables dans l'invention, on peut citer les huiles hydrocarbonées d'origine minérale ou synthétique telles que les hydrocarbures linéaires ou ramifiés comme l'huile de paraffine ou ses dérivés, l'huile de vaseline, les polydécènes, le polyisobutène hydrogéné tel que le Parléam commercialisé par la société NIPPON OIL FATS, le squalane d'origine synthétique ou végétale ; les huiles d'origine animale comme l'huile de vison, de tortue, le perhydrosqualène ; les huiles d'origine végétale hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées, notamment les triglycérides d'acide gras notamment de 4 à 22 atomes de carbone, comme les triglycérides des acides heptanoïque, octanoïque, et des acides caprique/caprylique ou bien encore les triglycérides hydroxylés, tels que l'huile d'amande douce, de calophyllum, de palme, de pépins de raisin, de sésame, d'arara, de colza, de tournesol, de coton, d'abricot, de ricin, de luzerne, de courge, de cassis, de macadamia, de rosier muscat, de noisette, d'avocat, de jojoba, d'olive, de germes de céréales (maïs, blé, orge, seigle), de beurre de karité ; des esters d'acides gras, en particulier de 4 à 22 atomes de carbone, et notamment d'acide octanoïque, d'acide heptanoïque, d'acide lanolique, d'acide oléique, d'acide laurique, d'acide stéarique comme le dioctanoate de propylène glycol, le monoisostéarate de propylène glycol, le polyglycéryl 2 diisostéarate, le diheptanoate de néopentylglycol ; les esters de synthèse de formule R₁COOR₂ dans laquelle R₁ représente le reste d'un acide gras supérieur linéaire ou ramifié comportant de 7 à 40 atomes de carbone et R₂ représente une chaîne hydrocarbonée ramifiée contenant de 3 à 40 atomes de carbone, comme par exemple l'huile de Purcellin (octanoate de cétostéaryle), l'isononanoate d'isononyle, le néopentanoate d'isodécyle, le benzoate d'alcool en C₁₂ à C₁₅, le palmitate d'éthyl 2-hexyle, le stéarate d'octyl-2 dodécyle, l'érucate d'octyl-2 dodécyle, l'isostéarate d'isostéaryle, le benzoate d'octyl-2 dodécyle, des octanoates, décanoates ou ricinoléates d'alcools ou de polyalcools, le myristate d'isopropyle, le palmitate d'isopropyle, le stéarate de butyle, le laurate d'hexyle, l'adipate de diisopropyle, le palmitate de 2-éthyl-hexyle, le laurate de 2-hexyl-décyle, le palmitate de 2-octyl-décyle, le myristate de 2-octyldodécyle, le succinate de 2-diéthyl-hexyle, le malate de diisostéaryle, le triisostéarate de glycérine ou de diglycérine ; les esters hydroxylés comme le lactate d'isostéaryle, l'octylhydroxystéarate, l'hydroxystéarate d'octyldodécyle, le diisostéarylmalate, le citrate de triisocétyle ; le diisononanoate de diéthylèneglycol ; les esters du pentaérythritol ; les esters d'acides aromatiques et d'alcools comprenant 4 à 22 atomes de carbone, notamment le trimellitate de tridécyle ; les acides gras supérieurs en C₈-C₂₆ tels que l'acide oléique, l'acide linoléique, l'acide linolénique ou l'acide isostéarique ; les alcools gras supérieurs en C₈-C₂₆ tels que l'alcool oléique, l'alcool linoléique ou linolénique, l'alcool isostéarique ou l'octyl dodécanol ; les éthers de synthèse à au moins 7 atomes de carbone, les huiles siliconées telles que les polydiméthylsiloxanes (PDMS) liquides à température ambiante, linéaires, éventuellement phénylés tels que les phényltriméthicones, les phényl triméthylsiloxy diphénylsiloxanes, les diphényl diméthicones, les diphényl méthyldiphényl trisiloxanes, les 2-phényléthyl triméthylsiloxysilicates liquides, éventuellement substitués par des groupements aliphatiques et/ou aromatiques comme les groupes alkyle, alkoxy ou phényle, pendant et/ou en bout de chaîne siliconée, groupements ayant de 2 à 24 atomes de carbone et éventuellement fluorés, ou par des groupements fonctionnels tels que des groupements hydroxyle, thiol et/ou aminé ; les polysiloxanes modifiés par des acides gras, des alcools gras ou des polyoxyalkylènes comme les diméthicones copolyols ou les alkylméthicones copolyols ; les silicones fluorées liquides ; ou encore les triglycérides des acides caprylique/caprique comme ceux vendus par la société STEARINERIES Dubois ou ceux vendus sous les dénominations Miglyol 810, 812 et 818 par la société DYNAMIT NOBEL, et leurs mélanges.

Selon un mode particulier de réalisation, l'huile non volatile autres que l'ester de dipentaérythritol, peut être choisie parmi le palmitate d'éthyle-2 hexyle, le néopentanoate d'isodécyle, le carbonate de di capryle, le stéarate d'octyl-2 dodécyle et leur mélange.

La composition peut comprendre une ou plusieurs huiles non volatiles en une teneur allant de 0,1 à 80 % en poids, de préférence de 1 à 50 % en poids et de manière encore plus préférentielle de 2 à 30 % en poids par rapport au poids total de la composition.

La phase grasse de la composition selon l'invention peut également comprendre au moins une cire, au moins une gomme et/ou au moins un corps gras pâteux, d'origine végétale, animale, minérale ou de synthèse, siliconé ou non.

Par « cire » au sens de la présente invention, on entend un composé gras lipophile, solide à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.
Les cires, au sens de la présente invention, peuvent être hydrocarbonées, siliconées et/ou fluorées, comportant éventuellement des fonctions ester ou hydroxyle. Elles sont notamment d'origine naturelle comme la cire d'abeilles éventuellement modifiée, la cire de Carnauba, de Candellila, d'Ouricoury, du Japon, de fibres de liège ou de canne à sucre, la cérésine, les cires de paraffine, de lignite, les cires microcristallines, la cire de lanoline, la cire de Montan, les ozokérites, les huiles hydrogénées comme l'huile de jojoba hydrogénée ou copolymérisation de l'éthylène, les cires obtenues par synthèse de Fischer-Tropsch, les esters d'acides gras et les glycérides concrets à 45 °C, les cires de silicone comme les alkyle, alkoxy et/ou esters de poly(di)méthylsiloxane solide à 45 °C, ayant de 10 à 45 atomes de carbone, certains acides gras comme l'acide stéarique, myristique, ou béhénique, et leurs mélanges.

La cire peut représenter de 0,1 à 30 % en poids, notamment de 0,5 à 20 % en poids, par rapport au poids total de la composition. Selon un mode de réalisation, la composition peut être exempte de cires.

Par « composé pâteux » au sens de l'invention, on entend un composé ayant un point de fusion allant de 25 à 60 °C, de préférence de 30 à 45 °C et une dureté allant de 0,001 à 0,5 MPa, de préférence de 0,005 à 0,4 MPa.
A titre d'exemple de corps gras pâteux, on peut citer les PDMS ayant des chaînes pendantes du type alkyle ou alkoxy ayant de 8 à 24 atomes de carbone comme le stéaryl diméthicone et notamment ceux vendus par DOW CORNING sous les références DC2503 ou DC05514 ; les esters d'alcool gras ou d'acide gras ayant de 20 à 25 atomes de carbone (un point de fusion notamment de 20 à 35 °C et/ou une viscosité à 40 °C allant de 0,1 à 40 Pa.s) comme les esters du cholestérol tels que les triglycérides d'origine végétale hydrogénés comme par exemple l'huile de ricin hydrogénée vendue sous le nom « Thixin R » par la société RHEOX, le polylaurate de vinyle, le propionate d'arachidyle, le triisostéaryl or cétyl citrate, le copolymère PVP/Eicosène ; les lanolines d'isopropyle, ayant une viscosité de 10 à 25 Pa.s, de préférence de 19 à 25 Pa.s et/ou un point de fusion de 25 à 45 °C et leurs mélanges.
La composition de l'invention peut également comprendre au moins une alkyl, alkoxy ou phényldiméthicone telle que, par exemple le produit vendu sous la dénomination de « Abil wax 2440® » par la société GOLDSCHMIDT.

### Phase aqueuse

La composition selon l'invention peut comprendre au moins une phase aqueuse contenant de l'eau.
L'eau peut être une eau florale telle que l'eau de bleuet et/ou une eau minérale telle que l'eau de VITTEL, l'eau de LUCAS ou l'eau de LA ROCHE POSAY et/ou une eau thermale.

Cette phase aqueuse peut également comprendre des solvants organiques miscibles à l'eau (25 °C) comme par exemple les alcools primaires tels que l'éthanol et l'isopropanol, les glycols tels que le glycérol, le propylène glycol, le butylène glycol, le dipropylène glycol, le diéthylène glycol, les éthers de glycol, les alkyl en C₁-C₄ éthers de mono-, di- ou tri- propylène glycol, mono-, di- ou tri- éthylène glycol, et leurs mélanges.

La phase aqueuse peut comprendre en outre des agents de stabilisation, par exemple le chlorure de sodium, le dichlorure de magnésium et le sulfate de magnésium.

Elle peut également incorporer tout composé hydrosoluble ou hydrodispersible compatible avec une phase aqueuse tels que des gélifiants, des polymères filmogènes, des épaississants, des tensioactifs et leurs mélanges.

Selon un mode de réalisation particulier, la phase aqueuse, et notamment l'eau, peut être présente dans la composition selon l'invention en une teneur allant de 1 à 60 % en poids, notamment de 5 à 50 % en poids, par rapport au poids total de la composition.

### Tensioactifs

Lorsque la composition selon l'invention se présente sous forme d'émulsion à phase continue huileuse (E/H), elle peut comprendre au moins un tensioactif émulsionnant.

On peut citer par exemple comme tensioactif émulsionnant, les esters gras de polyol, notamment de glycérol, de sorbitol, ou de polyéthylène glycol, et notamment les esters isostéariques, oléiques et ricinoléiques de polyol, tels que le mélange de petrolatum, d'oléate de polyglycéryl-3, d'isostéarate de glycéryle, le mélange de stéarate de glycéryl et de stéarate de PEG-100, vendu sous la denomination Arlacel 165 de la société ICI, l'huile de ricin hydrogénée et d'ozokérite, vendu sous la dénomination PROTEGIN W® par la société Goldschmidt, l'isostéarate de sorbitan, le di-isostéarate de polyglycéryle, le sesqui-isostéarate de polyglycéryle-2 ; les esters et éthers d'oses tels que le « Methyl glucose dioleate » ; les esters gras tels que le lanolate de magnésium ; les dimethicone copolyols et alkyl-dimethicone copolyols tels que le Laurylmethicone copolyol vendu sous la dénomination DOW CORNING 5200 FORMULATION AID par la société Dow Corning et le cétyl diméthicone copolyol vendu sous la dénomination ABIL EM 90® par la société Goldschmidt, le mélange de stéarate d'éthylène glycol acétyle et de tristéarate de glycéryle commercialisé par la société Guardian sous la dénomination commerciale UNITWIX, le copolymère de polyéthylène glycol (30 OE) et d'acide hydroxy -12 stéarique vendu sous la référence ARLACEL P 135 par la société Uniqema et leurs mélanges.

Les tensioactifs émulsionnants peuvent être introduits tels quels ou sous forme de mélange avec d'autres tensioactifs émulsionnants et/ou avec d'autres composés tels que des alcools gras ou des huiles.

On peut aussi utiliser comme émulsionnants, des polymères amphiphiles tels que les copolymères acryliques modifiés, comme par exemple les produits commercialisés sous les dénominations Pemulen par la société Goodrich ; les copolymères d'acide 2-acrylamido-2-méthylpropane sulfonique à chaîne hydrophobe, tels que décrits dans le document EP-A-1 069 142; les polyoléfines à terminaison succinique éventuellement estérifiée ou amidifiée, comme les composés décrits dans les documents US-A-4,234,435, US-A-4,708,753, US-A-5,129,972, US-A-4,931,110, GB-A-2,156,799 et US-A-4,919,179. Comme polyoléfines à terminaison succinique, on peut citer notamment les polyisobutylènes à terminaison succinique modifiée, tels que les produits commercialisés sous les dénominations L2724 et L2721 par la société Lubrizol.

La quantité de tensioactif émulsionnant peut aller de 0,1 à 10 % en poids, par rapport au poids total de la composition, et notamment de 1 à 5 % en poids.

### Epaississant

L'émulsion selon l'invention peut comprendre, en outre, un agent épaississant de la phase grasse. L'agent épaississant peut être choisi parmi :
- les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX,
- la silice pyrogénée hydrophobe, qui est une silice pyrogénée modifiée chimiquement en surface par réaction chimique générant une diminution du nombre de groupes silanol. On peut notamment substituer des groupes silanol par des groupements hydrophobes.
Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812®" par la société DEGUSSA, "CAB-O-SIL TS-530®" par la société CABOT,
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6^{ème} édition, 1995). Elles sont par exemple commercialisées sous les références "AEROSIL R972®", "AEROSIL R974®" par la société DEGUSSA, "CAB-O-SIL TS-610®", "CAB-O-SIL TS-720®" par la société CABOT.

La silice pyrogénée présente de préférence une taille de particules pouvant être nanométrique à micrométrique, par exemple allant d'environ de 5 à 200 nm.

L'agent épaississant de la phase grasse peut être présent en une teneur allant de 0,1 à 10 % en poids, et notamment de 1 à 5 % en poids par rapport au poids total de la composition.

### Phase particulaire

La phase particulaire de la composition selon l'invention peut comprendre, en outre, au moins une charge additionnelle, différente des particules de polymère acrylique.

Par « charge », on entend toute particule incolore choisie parmi les charges minérales ou organiques, lamellaires, sphériques ou oblongues, chimiquement inerte dans la composition.
On peut citer le talc, le mica, la silice, le kaolin, les poudres de polyamide comme le Nylon®, les poudres de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile telles que l'Expancel® (NOBEL INDUSTRIE), les poudres de polyuréthane, les microbilles de résine de silicone (Tospearls® de TOSHIBA, par exemple), le carbonate de calcium précipité, le dicalcium phosphate, le carbonate ou l'hydrocarbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges. Ces charges peuvent être traitées ou non en surface notamment pour les rendre lipophiles.

Les charges peuvent être présentes dans la composition selon l'invention en une teneur allant de 0,1 à 30 % en poids, de préférence de 0,5 à 20 % en poids et de manière encore plus préférentielle de 1 à 10 % par rapport au poids total de la composition.

### Matières colorantes

La composition peut contenir avantageusement au moins une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,001 à 40 % en poids, notamment de 0,1 à 30 % en poids, en particulier de 2 à 25 % en poids, et plus particulièrement de 3 à 20 % en poids par rapport au poids total de la composition.

Les colorants lipophiles, synthétiques ou naturels peuvent, par exemple, être choisis parmi le DC Red 17, le DC Red 21, le DC Red 27, le DC Green 6, le DC Yellow 11, le DC Violet 2, le DC Orange 5, le rouge Soudan, les carotènes (le β-carotène, le lycopène), les xanthophylles (capsanthine, capsorubine, lutéine), l'huile de palme, le brun Soudan, le jaune quinoléine, le rocou, le curcumin.

Les colorants hydrosolubles synthétiques ou naturels peuvent, par exemple, être choisis parmi le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5, le FDC Blue 1, la bétanine (betterave), le carmin, la chlorophylline cuivrée, le bleu de méthylène, les anthocyanines (enocianine, carotte noire, hibiscus, sureau), le caramel, la riboflavine.

Les colorants peuvent encore être choisis parmi la juglone, la lawsone, les extraits de soja fermenté, d'algues, de champignons, de micro-organismes. Les sels de Flavylium non substitués en position 3 tels que par exemple ceux décrits dans le brevet EP 1 172 091, les extraits de Gesneria Fulgens, Blechum Procerum, Saxifraga et les pigments susceptibles d'être obtenus par extraction par un solvant organique ou hydro-organique d'un milieu de culture de micromycètes du type monascus Monascus.

Les colorants peuvent représenter de 0,001 à 3 % en poids, et en particulier de 0,01 à 1 % en poids, par rapport au poids total de la composition.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D&C, et les laques à base de carmin de cochenille, de baryum, strontium, calcium, aluminium. Les pigments peuvent représenter de 0,1 à 40 % en poids, notamment de 1 à 35 % en poids, et en particulier de 2 à 25 % en poids, par rapport au poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0,1 à 20 % en poids, et en particulier de 0,1 à 15 % en poids, par rapport au poids total de la composition.

Avantageusement, l'émulsion selon l'invention comprend des pigments enrobés hydrophobes. Les pigments enrobés hydrophobes sont des pigments (tels que ceux cités précédemment) traités en surface avec un agent hydrophobe pour les rendre compatibles avec la phase grasse de l'émulsion, notamment pour qu'ils aient une bonne mouillabilité avec les huiles de la phase grasse de manière à ce que ces pigments traités soient bien dispersés dans la phase grasse.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine.

Le terme « alkyle » mentionné dans les composés agents hydrophobes cités précédemment désigne notamment un groupe alkyle ayant de 1 à 30 atomes de carbone, de préférence ayant de 5 à 16 atomes de carbone.

Des pigments traités hydrophobes sont notamment décrits dans la demande EP-A-1086683.

### Additifs

La composition de l'invention peut comprendre, en outre, tout additif complémentaire usuellement utilisé dans le domaine concerné, tel que des antioxydants, des conservateurs, des neutralisants, des gélifiants lipophiles, des gélifiants de phase aqueuse, des dispersants, des actifs cosmétiques, des parfums, des agents anti-radicaux libres, des séquestrants, des agents filmogènes, et leurs mélanges. Ces additifs peuvent être présents dans la composition à raison de 0,0005 à 20 % en poids, et en particulier de 0,001 à 10 % en poids, par rapport au poids total de la composition.

Comme actif cosmétique utilisable dans l'invention, on peut citer les vitamines A, E, C, B₃, F, les provitamines comme le D-panthénol, les actifs apaisants comme l'a-bisabolol, l'aloe vera, l'allantoïne, les extraits de plantes ou les huiles essentielles, les agents protecteurs ou restructurants comme les céramides, les actifs « fraîcheur » comme le menthol et ses dérivés, les émollients, les hydratants, les actifs antirides, les acides gras essentiels, les filtres solaires, et leurs mélanges.

Bien entendu, l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.
La composition selon l'invention peut être fabriquée par les procédés connus, généralement utilisés dans le domaine cosmétique.

La composition selon l'invention présente une phase continue huileuse, et peut notamment se présenter sous forme de produit anhydre ou d'émulsion eau-dans-huile (E/H) ou encore d'émulsion triple huile-dans-eau-dans-huile (H/E/H). Selon un mode préféré de réalisation, la composition se présente sous forme d'émulsion E/H.

La composition selon l'invention peut être une composition de maquillage de la peau, comme un fond de teint, un produit anti-cernes, une crème teintée, une composition de maquillage du corps, et en particulier un fond de teint à appliquer sur le visage et/ou le cou.

L'invention est illustrée plus en détails dans les exemples ci-après.

### Exemples

Les exemples figurant ci-après sont présentés à titre illustratif et non limitatif au domaine de l'invention.

### Exemples 1 à 10 de fonds de teint

On a préparé les compositions de fond de teint suivantes :

| | | % en poids |
|---|---|---|
| **A1** | Copolymère de polyéthylène glycol (30 OE) et d'acide 12-hydroxy stéarique vendu sous la référence ARLACEL P 135 par la Société UNIQEMA | 4,00 |
| | Palmitate d'éthyle-2 hexyle | 2,00 |
| | Mélange de tri-stéarate de glycérine et de stéarate d'éthylène glycol acétylé vendu sous la référence UNITWIX par la société UNITED GUARDIAN | 0,70 |
| | Dicapryl carbonate | 3,60 |
| | Stéarate d'octyle-2 dodécyle | 4,00 |
| | Para-méthoxy cinnamate d'éthyle-2 hexyle | 5,00 |
| | Isohéxadécane | 3,60 |
| | **HUILE** | 10,00 |
| | Conservateur | 0,15 |
| **A2** | Gel de bentone vendu sous la référence BENTONE GEL IHD V par la société ELEMENTIS | 4,00 |
| **A3** | Néopentanoate d'isodécyle | 4,20 |
| | Oxyde de fer jaune enrobé de stéaroyl glutamate d'aluminium | 1,80 |
| | Oxyde de fer brun enrobé de stéaroyl glutamate d'aluminium | 0,49 |
| | Oxyde de fer noir enrobé de stéaroyl glutamate d'aluminium | 0,21 |
| | Dioxyde de titane enrobé de stéaroyl glutamate d'aluminium | 7,50 |
| **A4** | **CHARGE** | **5,00** |
| **B** | Eau déminéralisée | 37,15 |
| | Conservateur | 0,40 |
| | Sulfate de magnésium | 0,60 |
| | EDTA | 0,10 |
| | Octane 1-2 diol | 0,50 |
| | Glycérol | 5,00 |
| | TOTAL | 100% |

### Mode opératoire

On pèse les constituants de la phase A1 dans le bécher principal et on le place au bain-marie (85°C).
Lorsque le mélange est homogène, on ajoute A2 sous agitation au Moritz à 1500 tours / min.
La phase A3 est préparée séparément en broyant les pigments à la tri-cylindre puis elle est ajoutée sous agitation au mélange précédent.

Puis on incorpore la phase A4 en maintenant la même agitation.
Pour préparer la phase aqueuse B, on pèse l'eau, le glycérol et le conservateur que l'on porte à l'ébullition jusqu'à dissolution. Puis on incorpore le sulfate de magnésium et l'EDTA puis enfin l'octane 1, 2 diol à 45 °C.
L'émulsion se fait à température ambiante : on verse la phase aqueuse dans la phase grasse en augmentant au fur et à mesure la vitesse d'agitation du Moritz jusqu'à 4000-4500 tours / min. Après ajout de la phase aqueuse, on laisse sous agitation pendant encore 10 min.

### Influence de la nature de la charge :

On a comparé l'influence de la nature de la charge sur les propriétés des fonds de teint selon l'invention. Pour cela, on a choisi l'héxacaprylate/caprate de dipentaérythrityle vendu sous la dénomination de DUB DPHCC par la société STEARINERIE DUBOIS comme huile dans la composition exemplifiée précédemment.

Les charges testées sont les suivantes :

| | **CHARGE** |
|---|---|
| EX1 (invention) | Copolymère méthyl méthacrylate/ éthylène glycol di méthacrylate vendu sous la référence TECHPOLYMER MBP-8 par la société SEKISUI PLASTICS |
| EX2 (comparaison) | Nylon 12 vendu sous la référence ORGASOL 2002 EXD NAT COS par la société ARKEMA |
| EX3 (comparaison) | Polyméthylsilsesquioxane vendu sous la référence TOSPEARL 145 A par la société GE TOSHIBA SILICONES |
| EX4 (comparaison) | Silice vendue sous la référence SUNSPHERE H51 par la société ASAHI GLASS SI-TECH |

Les propriétés cosmétiques des compositions obtenues ont été comparées par un panel de 5 expertes.
Pour chaque test, on a maquillé un demi visage avec une quantité de 0,15 g de fond de teint selon l'exemple 1 (invention) et l'autre demi-visage par une composition selon les exemples 2, 3 ou 4 (hors invention).

L'exemple 1 (selon l'invention) comparativement au fond de teint selon l'exemple 2 (hors invention) forme un film moins collant une fois appliqué sur la peau.
En comparaison avec les exemples 3 et 4 (hors invention), le fond de teint selon l'exemple 1 (invention) procure un résultat maquillage moins gras.

### Influence de la nature de l'huile :

On a comparé l'influence de la nature de l'huile sur les propriétés des fonds de teint selon l'invention. Pour cela, on a choisi copolymère méthacrylate de méthyle / bis méthacrylate d'éthylène glycol vendu sous la référence TECHPOLYMER MBP-8 par la société SEKISUI PLASTICS comme charge dans la composition exemplifiée précédemment.

Les huiles testées sont les suivantes :

| | **HUILE** |
|---|---|
| EX1 (invention) | Héxacaprylate/caprate de dipentaérythrityle vendu sous la référence DUB DPHCC par la société STEARINERIE DUBOIS |
| EX5 (comparaison) | Tétra caprylate/caprate de pentaérythrityle vendu sous la référence DUB PTCC par la société STEARINERIE DUBOIS |
| EX6 (comparaison) | Tétra éthyl-2 héxanoate de pentaérythrityle vendu sous la référence TRIVENT PE 48 par la société ALZO |
| EX7 (comparaison) | Tétra héxyl-2 décanoate de pentaérythrityle vendu sous la référence ISOCARB ESTER 1605 par la société SASOL |
| EX8 (comparaison) | Tétra isostéarate de pentaérythrityle vendu sous la référence PRISORINE 3631 par la société UNIQEMA |
| EX9 (comparaison) | Tri isostéarate de glycéryle vendu sous la référence DUB TGIS par la société STEARINERIE DUBOIS |
| EX10 (comparaison) | Tri décyl -2 tétradécanoate de glycéryle vendu sous la référence DUB TGI 24 par la société STEARINERIE DUBOIS |

Les propriétés cosmétiques des compositions obtenues ont été comparées par un panel de 5 expertes.
Pour chaque test, on a maquillé un demi visage avec une quantité de 0,15 g de fond de teint selon l'exemple 1 (invention) et l'autre demi-visage par une composition selon les exemples 5 à 10 (hors invention).

Le fond de teint selon l'exemple 1 (invention), comparé aux fonds de teint selon les exemples 5 à 10 (hors invention), forme un film moins gras une fois appliqué sur la peau. Le fini maquillage est plus poudré.
En comparaison avec les exemples 5 et 9 (hors invention), l'exemple 1 permet de former un film de maquillage moins collant une fois appliqué sur la peau.

### Exemple 11 de fond de teint

On a préparé un fond de teint ayant la composition suivante :

| | | % en poids |
|---|---|---|
| **A1** | Copolymère de polyéthylène glycol (30 OE) et d'acide 12-hydroxy stéarique vendu sous la référence ARLACEL P 135 par la Société UNIQEMA | 4,00 |
| | Palmitate d'éthyle-2 hexyle | 2,80 |
| | Mélange de tri-stéarate de glycérine et de stéarate d'éthylène glycol acétylé vendu sous la référence UNITWIX par la société UNITED GUARDIAN | 0,70 |
| | Dicapryl carbonate | 5,00 |
| | Stéarate d'octyle-2 dodécyle | 5,60 |
| | Para-méthoxy cinnamate d'éthyle-2 hexyle | 5,00 |
| | Isohéxadécane | 5,00 |
| | Hexa C5-9 carboxylate de dipentaérythrityle vendu sous la dénomination de LexFeel 350 par la société INOLEX CHEMICAL COMPANY | 3,00 |
| | Conservateur | 0,15 |
| **A2** | Gel de bentone vendu sous la référence BENTONE GEL IHD V par la société ELEMENTIS | 4,00 |
| **A3** | Néopentanoate d'isodécyle | 6,00 |
| | Oxyde de fer jaune enrobé de stéaroyl glutamate d'aluminium | 1,80 |
| | Oxyde de fer brun enrobé de stéaroyl glutamate d'aluminium | 0,49 |
| | Oxyde de fer noir enrobé de stéaroyl glutamate d'aluminium | 0,21 |
| | Dioxyde de titane enrobé de stéaroyl glutamate d'aluminium | 7,50 |
| **A4** | Copolymère méthyl méthacrylate/ Ethylene glycol di methacrylate vendu sous la référence TECHPOLYMER MBP-8 par la société SEKISUI PLASTICS | 5,00 |
| **B** | Eau déminéralisée | 37,15 |
| | Conservateur | 0,40 |
| | Sulfate de magnésium | 0,60 |
| | EDTA | 0,10 |
| | Octane 1-2 diol | 0,50 |
| | Glycérol | 5,00 |
| | TOTAL | 100% |

Le mode opératoire est le même que pour les exemples 1 à 10.

On obtient un fond de teint présentant de bonnes propriétés de douceur et d'étalement lors de l'application sur la peau et qui ne laisse pas une sensation de gras voire de collant à l'application ou au cours de la journée.

## Revendications

1. Composition de maquillage ou de soin de la peau comprenant une phase continue huileuse et une phase particulaire, ladite phase continue huileuse comprenant au moins un ester de dipentaérythritol obtenu par estérification totale du dipentaérythritol par des acides linéaires ou ramifiés, saturés ou insaturés comportant de 3 à 24 atomes de carbone, de préférence de 4 à 18 atomes de carbone et de manière encore plus préférentielle de 5 à 16 atomes de carbone, et ladite phase particulaire comprenant des particules de polymère acrylique.

2. Composition selon la revendication précédente, **caractérisée en ce que** les particules de polymère acrylique ne sont pas filmogènes.

3. Composition selon l'une quelconque des revendications précédentes, **caractérisée en ce que** les particules de polymère acrylique sont choisies parmi :
- les particules de polyméthylméthacrylate réticulé ou non réticulé,
- les particules de copolymère méthacrylate de méthyle / acrylate de butyle,
- les particules de copolymère acrylate de méthyle / éthylène ;
- les particules de copolymère réticulé méthacrylate de méthyle / di méthacrylate d'éthylène glycol,
- les particules de polyméthacrylate de méthyle / di méthacrylate d'éthylène glycol,
- les particules de copolymère de polyméthacrylate d'allyle/di méthacrylate d'éthylène glycol,
- les particules de copolymère méthacrylate de lauryle / di méthacrylate d'éthylène glycol.

4. Composition selon l'une quelconque des revendications précédentes, **caractérisée en ce que** les particules de polymère acrylique sont présentes en une quantité allant de 0,1 à 10 % en poids, par rapport au poids total de la composition, en particulier de 2 à 7 % et plus particulièrement de 3 à 6% en poids.

5. Composition selon l'une quelconque des revendications précédentes, **caractérisée en ce que** l'ester de dipentaérythritol est l'héxacaprylate/caprate de dipentaérythrityle,

6. Composition selon l'une quelconque des revendications précédentes, **caractérisée en ce que** l'ester de dipentaérythritol est présent en une quantité allant de 0,1 à 30% en poids par rapport au poids total de la composition, en particulier de 1 à 20% et plus particulièrement de 3 à 15% en poids.

7. Composition selon l'une quelconque des revendications précédentes, **caractérisée en ce que** la phase huileuse comprend au moins une huile additionnelle, volatile ou non volatile.

8. Composition selon la revendication précédente, **caractérisée en ce que** l'huile volatile additionnelle est l'isohéxadécane et est présente en une teneur allant de 1 % à 80 % en poids par rapport au poids total de la composition, notamment allant de 2 % à 50 % en poids, et mieux de 3 à 30% en poids.

9. Composition selon la revendication 7, **caractérisée en ce que** l'huile non volatile est choisie parmi le palmitate d'éthyle-2 hexyle, le néopentanoate d'isodécyle, le carbonate de di capryle et leur mélange.

## Patentansprüche

1. Zusammensetzung zum Schminken und Pflegen der Haut, umfassend eine kontinuierliche Ölphase und eine Partikelphase, wobei die kontinuierliche Ölphase mindestens ein Dipentaerythritolester umfasst, das durch totale Veresterung des Dipentaerythritol durch lineare oder verzweigte, gesättigte oder ungesättigte Säuren, umfassend 3 bis 24 Kohlenstoffatome, vorzugsweise 4 bis 18 Kohlenstoffatome und noch bevorzugter 5 bis 16 Kohlenstoffatome, erhalten wird, und wobei die Partikelphase Acrylpolymerpartikel umfasst.

2. Zusammensetzung nach dem vorhergehenden Anspruch, **dadurch gekennzeichnet, dass** die Acrylpolymerpartikel nicht filmbildend sind.

3. Zusammensetzung nach einem der vorhergehenden Ansprüche, **dadurch gekennzeichnet, dass** die Acrylpolymerpartikel ausgewählt sind unter:
- den Partikeln von vernetztem oder nicht vernetztem Polymethylmethacrylat,
- den Partikeln eines Copolymers Methylmethacrylat / Butylacrylat,
- den Partikeln eines Copolymers Methylacrylat / Ethylen,
- den Partikeln eines vernetzten Copolymers Methylmethacrylat / Ethylenglycoldimethacrylat,
- den Partikeln von Methylpolymethacrylat / Ethylenglycoldimethacrylat,
- den Partikeln eines Copolymers von Allylpolymethacrylat / Ethylenglycoldimethacrylat,
- den Partikeln eines Copolymers von Laurylmethacrylat / Ethylenglycoldimethacrylat.

4. Zusammensetzung nach einem der vorhergehenden Ansprüche, **dadurch gekennzeichnet, dass** die Acrylpolymerpartikel in einer Menge von 0,1 bis 10 Gew.-% bezogen auf das Gesamtgewicht der Zusammensetzung, insbesondere von 2 bis 7 Gew.-% und noch bevorzugter von 3 bis 6 Gew.-%, vorhanden sind.

5. Zusammensetzung nach einem der vorhergehenden Ansprüche, **dadurch gekennzeichnet, dass** das Dipentaerythritolester das Hexacaprylat / Dipentaerythritylcaprat ist.

6. Zusammensetzung nach einem der vorhergehenden Ansprüche, **dadurch gekennzeichnet, dass** das Dipentaerythritolester in einer Menge von 0,1 bis 30 Gew.-% bezogen auf das Gesamtgewicht der Zusammensetzung, insbesondere von 1 bis 20 Gew.-% und noch bevorzugter von 3 bis 15 Gew.-%, vorhanden ist.

7. Zusammensetzung nach einem der vorhergehenden Ansprüche, **dadurch gekennzeichnet, dass** die Ölphase mindestens ein zusätzliches, flüchtiges oder nicht flüchtiges Öl umfasst.

8. Zusammensetzung nach dem vorhergehenden Anspruch, **dadurch gekennzeichnet, dass** das zusätzliche flüchtige Öl das Isohexadecan ist, und einen Gehalt von 1 bis 80 Gew.-% bezogen auf das Gesamtgewicht der Zusammensetzung, insbesondere von 2 bis 50 Gew.-% und besser von 3 bis 30 Gew.-%, aufweist.

9. Zusammensetzung nach Anspruch 7, **dadurch gekennzeichnet, dass** das nicht flüchtige Öl ausgewählt ist unter Ethyl-2 Hexylpalmitat, Isodecylneopentanoat, Dicaprylcarbonat und ihrer Mischung.

## Claims

1. Composition for making up or caring for the skin, comprising an oily continuous phase and a particulate phase, said oily continuous phase comprising at least one dipentaerythritol ester obtained by complete esterification of dipentaerythritol with linear or branched, saturated or unsaturated acids containing from 3 to 24 carbon atoms, preferably from 4 to 18 carbon atoms, and even more preferably from 5 to 16 carbon atoms, and said particulate phase comprising particles of acrylic polymer.

2. Composition according to the preceding claim, **characterized in that** the particles of acrylic polymer are not film-forming.

3. Composition according to any one of the preceding claims, **characterized in that** the particles of acrylic polymer are chosen from:
- particles of crosslinked or noncrosslinked polymethyl methacrylate,
- particles of methyl methacrylate/butyl acrylate copolymer,
- particles of methyl acrylate/ethylene copolymer,
- particles of methyl methacrylate/ethylene glycol dimethacrylate crosslinked copolymer,
- particles of polymethyl methacrylate/ethylene glycol dimethacrylate,
- particles of polyallyl methacrylate/ethylene glycol dimethacrylate copolymer,
- particles of lauryl methacrylate/ethylene glycol dimethacrylate copolymer.

4. Composition according to any one of the preceding claims, **characterized in that** the particles of acrylic polymer are present in an amount ranging from 0.1% to 10% by weight, relative to the total weight of the composition, in particular from 2% to 7%, and more particularly from 3% to 6% by weight.

5. Composition according to any one of the preceding claims, **characterized in that** the dipentaerythritol ester is dipentaerythrityl hexacaprylate/caprate.

6. Composition according to any one of the preceding claims, **characterized in that** the dipentaerythritol ester is present in an amount ranging from 0.1% to 30% by weight, relative to the total weight of the composition, in particular from 1% to 20%, and more particularly from 3% to 15% by weight.

7. Composition according to any one of the preceding claims, **characterized in that** the oily phase comprises at least one additional volatile or non-volatile oil.

8. Composition according to the preceding claim, **characterized in that** the additional volatile oil is isohexadecane and is present at a content ranging from 1% to 80% by weight, relative to the total weight of the composition, in particular ranging from 2% to 50% by weight, and better still from 3% to 30% by weight.

9. Composition according to Claim 7, **characterized in that** the non-volatile oil is chosen from 2-ethylhexyl palmitate, isodecyl neopentanoate and dicapryl carbonate, and a mixture thereof.
